# EUROPEAN PATENT SPECIFICATION

(11) **EP 2 579 981 B1**
(45) Date of publication and mention of the grant of the patent: **07.06.2023**
(21) Application number: 10798661.4
(22) Date of filing: 13.12.2010
(51) Int. Cl.: B01J 21/06, B01J 21/08, B01J 23/52, B01J 21/16, B01J 21/18, B01J 35/00, B01J 37/02

(54) **CATALYST COMPRISING PLATINUM AND GOLD NANO-PARTICLES AND ITS USE FOR OXIDATION OF GLUCOSE**
KATALYSATOR MIT PLATIN- UND GOLDNANOPARTIKELN UND SEINE VERWENDUNG ZUR OXIDATION VON GLUCOSE
CATALYSEUR COMPRENANT DES NANOPARTICULES DE PLATINE ET D'OR ET SON UTILISATION POUR OXYDER LE GLUCOSE

(30) Priority: 11.06.2010 US 814188
(43) Date of publication of application: 17.04.2013
(60) Divisional application: 23170392.7
(73) Proprietor: Archer-Daniels-Midland Company, Chicago, IL 60601 (US)
(72) Inventor: MURPHY, Vincent J., Santa Clara, CA 95051 (US); SHOEMAKER, James, Santa Clara, CA 95051 (US); ZHU, Guang, Santa Clara, CA 95051 (US); ARCHER, Raymond, Santa Clara, CA 95051 (US); SALEM, George Frederick, Santa Clara, CA 95051 (US); DIAS, Eric L., Santa Clara, CA 95051 (US)
(74) Representative: Potter Clarkson
(86) International application number: PCT/US2010/060143
(87) International publication number: WO 2011/155964

(56) References cited:
- WO-A1-2005/003072
- WO-A1-2005/003072
- US-B1- 6 232 264
- US-B1- 6 232 264
- NIKOLAOS DIMITRATOS ET AL: "Synergetic effect of platinum or palladium on gold catalyst in the selective oxidation of D-sorbitol", CATALYSIS LETTERS, KLUWER ACADEMIC PUBLISHERS-PLENUM PUBLISHERS, NE, vol. 99, no. 3-4, 1 February 2005 (2005-02-01), pages 181-185, XP019275517, ISSN: 1572-879X
- YIHONG SHEN ET AL: "Efficient synthesis of lactic acid by aerob oxidation of glycerol on Au-Pt/TiO2 catalysts", CHEMISTRY - A EUROPEAN JOURNAL, WILEY - V C H VERLAG GMBH & CO. KGAA, WEINHEIM, DE, vol. 16, no. 25, 1 January 2010 (2010-01-01), pages 7368-7371, XP009146561, ISSN: 0947-6539 [retrieved on 2010-05-18]
- LAURA PRATI ET AL: "Effect of gold addition on Pt and Pd catalysts in liquid phase oxidations", TOPICS IN CATALYSIS, KLUWER ACADEMIC PUBLISHERS-PLENUM PUBLISHERS, NE, vol. 44, no. 1-2, 1 June 2007 (2007-06-01) , pages 319-324, XP019509110, ISSN: 1572-9028, DOI: DOI:10.1007/S11244-007-0305-9
- LORNA B ORTIZ-SOTO ET AL: "Structure-Sensitivity of Propylene Hydrogenation over Cluster-Derived Bimetallic Pt-Au Catalysts", CATALYSIS LETTERS, KLUWER ACADEMIC PUBLISHERS-PLENUM PUBLISHERS, NE, vol. 107, no. 1-2, 1 February 2006 (2006-02-01), pages 13-17, XP019275362, ISSN: 1572-879X
- CLARKE J K A ET AL: "Preparation of supported platinum-gold catalysts and alkane reactions on selected platinum and platinum-gold supported clusters", APPLIED CATALYSIS, AMSTERDAM, NL, vol. 9, no. 1, 1 January 1984 (1984-01-01) , pages 85-108, XP009146562, ISSN: 0166-9834
- BESSON M ET AL: "Oxidation of glucose and gluconate on Pt, Pt Bi, and Pt Au catalysts", RECUEIL DES TRAVAUX CHIMIQUES DES PAYS, ELSEVIER SCIENCE PUBLISHERS, AMSTERDAM, NL, vol. 115, no. 4, 1 January 1996 (1996-01-01), pages 217-221, XP008131910, ISSN: 0165-0513, DOI: 10.1002/RECL.19961150405
- NIKOLAOS DIMITRATOS ET AL: "Synergetic effect of platinum or palladium on gold catalyst in the selective oxidation of D-sorbitol", CATALYSIS LETTERS, KLUWER ACADEMIC PUBLISHERS-PLENUM PUBLISHERS, NE, vol. 99, no. 3-4, 1 February 2005 (2005-02-01), pages 181-185, XP019275517, ISSN: 1572-879X
- YIHONG SHEN ET AL: "Efficient synthesis of lactic acid by aerob oxidation of glycerol on Au-Pt/TiO2 catalysts", CHEMISTRY - A EUROPEAN JOURNAL, JOHN WILEY & SONS, INC, DE, vol. 16, no. 25 1 January 2010 (2010-01-01), pages 7368-7371, XP009146561, ISSN: 0947-6539, DOI: 10.1002/CHEM.201000740 [retrieved on 2010-05-18]
- LAURA PRATI ET AL: "Effect of gold addition on Pt and Pd catalysts in liquid phase oxidations", TOPICS IN CATALYSIS, KLUWER ACADEMIC PUBLISHERS-PLENUM PUBLISHERS, NE, vol. 44, no. 1-2, 1 June 2007 (2007-06-01) , pages 319-324, XP019509110, ISSN: 1572-9028, DOI: 10.1007/S11244-007-0305-9
- LORNA B ORTIZ-SOTO ET AL: "Structure-Sensitivity of Propylene Hydrogenation over Cluster-Derived Bimetallic Pt-Au Catalysts", CATALYSIS LETTERS, KLUWER ACADEMIC PUBLISHERS-PLENUM PUBLISHERS, NE, vol. 107, no. 1-2, 1 February 2006 (2006-02-01), pages 13-17, XP019275362, ISSN: 1572-879X
- CLARKE J K A ET AL: "Preparation of supported platinum-gold catalysts and alkane reactions on selected platinum and platinum-gold supported clusters", APPLIED CATALYSIS, AMSTERDAM, NL, vol. 9, no. 1, 1 January 1984 (1984-01-01) , pages 85-108, XP009146562, ISSN: 0166-9834, DOI: 10.1016/0166-9834(84)80040-8
- A. Habrioux ET AL: "Activity of Platinum−Gold Alloys for Glucose Electrooxidation in Biofuel Cells", The Journal of Physical Chemistry B, vol. 111, no. 34, 1 August 2007 (2007-08-01), pages 10329-10333, XP055027481, ISSN: 1520-6106, DOI: 10.1021/jp0720183
- KERZENMACHER S ET AL: "Energy harvesting by implantable abiotically catalyzed glucose fuel cells", JOURNAL OF POWER SOURCES, ELSEVIER, AMSTERDAM, NL, vol. 182, no. 1 15 July 2008 (2008-07-15), pages 1-17, XP022703970, ISSN: 0378-7753, DOI: 10.1016/J.JPOWSOUR.2008.03.031 [retrieved on 2008-03-22]

## Description

### FIELD OF THE INVENTION

The present invention relates to catalyst compositions comprising particles of gold-platinum alloy and particles of platinum (0) on a support selected from the group consisting of titanias, zirconias, silicas and montmorillonites, wherein (a) the molar ratio of platinum to gold on the support is from 10:1 to 1:2, and (b) the particle sizes of the platinum (0) particles are substantially in the range of from 5 to 30 nanometers and which are used for preparing glucaric acid comprising reacting glucose with a source of oxygen in the presence of the claimed catalyst composition and in the substantial absence of added base.

### BACKGROUND OF THE INVENTION

Crude oil is currently the source of most commodity and specialty organic chemicals. Many of these chemicals are employed in the manufacture of polymers and other materials. Examples include ethylene, propylene, styrene, bisphenol A, terephthalic acid, adipic acid, caprolactam, hexamethylene diamine, adiponitrile, caprolactone, acrylic acid, acrylonitrile, 1,6-hexanediol, 1,3-propanediol, and others. Crude oil is first refined into hydrocarbon intermediates such as ethylene, propylene, benzene, and cyclohexane. These hydrocarbon intermediates are then typically selectively oxidized using various processes to produce the desired chemical. For example, crude oil is refined into cyclohexane which is then selectively oxidized to "KA oil" which is then further oxidized for the production of adipic acid, an important industrial monomer used for the production of nylon 6,6. Many known processes are employed industrially to produce these petrochemicals from precursors found in crude oil. For example, see Ullmann's Encyclopedia of Industrial Chemistry, Wiley 2009 (7th edition).

For many years there has been interest in using biorenewable materials as a feedstock to replace or supplement crude oil. See, for example, Klass, Biomass for Renewable Energy, Fuels, and Chemicals, Academic Press, 1998. Moreover, there have been efforts to produce carboxylic acids from renewable resources using processes involving a combination of biocatalytic and chemocatalytic processes. See, for example, "Benzene-Free Synthesis of Adipic Acid", Frost et al. Biotechnol. Prog. 2002, Vol. 18, pp. 201-211, and U.S. Patent Nos. 4,400,468, and 5,487,987.

One of the major challenges for converting biorenewable resources such as carbohydrates (e.g., glucose derived from starch, cellulose or sucrose) to current commodity and specialty chemicals is the selective conversion of the primary alcohol (hydroxyl) group to a carboxyl group (COOH) in the presence of at least a secondary alcohol group.

Glucose can be obtained from various carbohydrate-containing sources including conventional biorenewable sources such as corn grain (maize), wheat, potato, cassava and rice as well as alternative sources such as energy crops, plant biomass, agricultural wastes, forestry residues, sugar processing residues and plant-derived household wastes. More generally, biorenewable sources include any renewable organic matter that includes a source of carbohydrates such as, for example, switch grass, miscanthus, trees (hardwood and softwood), vegetation, and crop residues (e.g., bagasse and corn stover). Other sources can include, for example, waste materials (e.g., spent paper, green waste, municipal waste, etc.).

Carbohydrates such as glucose may be isolated from biorenewable materials using methods that are known in the art. See, for example, Centi and van Santen, Catalysis for Renewables, Wiley-VCH, Weinheim 2007; Kamm, Gruber and Kamm, Biorefineries-Industrial Processes and Products, Wiley-VCH, Weinheim 2006; Shang-Tian Yang, Bioprocessing for Value-Added Products from Renewable Resources New Technologies and Applications, Elsevier B.V. 2007; Furia, Starch in the Food Industry, Chapter 8, CRC Handbook of Food Additives 2nd Edition CRC Press, 1973. See also chapters devoted to Starch, Sugar and Syrups within Kirk-Othmer Encyclopedia of Chemical Technology 5th Edition, John Wiley and Sons 2001. Also, processes to convert starch to glucose are known in the art, see, for example, Schenck, "Glucose and Glucose-containing Syrups" in Ullmann's Encyclopedia of Industrial Chemistry, Wiley-VCH 2009. Furthermore, methods to convert cellulose to glucose are known in the art, see, for example, Centi and van Santen, Catalysis for Renewables, Wiley-VCH, Weinheim 2007; Kamm, Gruber and Kamm, Biorefineries-Industrial Processes and Products, Wiley-VCH, Weinheim 2006; Shang-Tian Yang, Bioprocessing for Value-Added Products from Renewable Resources New Technologies and Applications, Elsevier B.V. 2007.

The selective oxidation of glucose to glucaric acid has been attempted by using oxidation methods that employ platinum catalysts. See, for example, U.S. Patent No. 2,472,168, which illustrates a method for the preparation of glucaric acid from glucose using a platinum catalyst in the presence of oxygen and a base. Further examples of the preparation of glucaric acid from glucose using a platinum catalyst in the presence of oxygen and a base are illustrated in the Journal of Catalysis Vol. 67, p. 1-13, and p. 14-20 (1981). Other oxidation methods have also been employed; see, for example, U.S. Patent Nos. 6,049,004, 5,599,977, and 6,498,269, WO 2008/021054 and J. Chem. Technol. Biotechnol. Vol. 76, p. 186-190 (2001); J. Agr. Food Chem. Vol. 1, p.779-783 (1953); J. Carbohydrate Chem. Vol. 21, p. 65-77 (2002); Carbohydrate Res. Vol. 337, p. 1059-1063 (2002); Carbohydrate Res. 336, p. 75-78 (2001); and Carbohydrate Res. Vol. 330, p. 21-29 (2001). However, these processes suffer from economic shortcomings resulting from, among other matters, process yield limitations, low conversion rates, and limited selectivity due to shortcomings in the performance of existing catalysts. None of these catalysts or the processes employing them are used industrially for the selective oxidation of glucose-containing carbohydrates to manufacture specialty or industrial carboxylic acids or derivatives thereof.

WO2005/003072 describes a process for the preparation of aldonic acids (in particular gluconic acid) by oxidation of aldoses with oxygen or with a gas containing oxygen, in the presence of supported bimetallic catalysts based on gold and platinum.

Thus, there remains a need for new, industrially scalable catalysts for the selective and commercially-meaningful conversion of a primary hydroxyl group to a carboxyl group of compositions comprising a primary hydroxyl group and at least a secondary hydroxyl group. Desirably, there is the need to convert biorenewable materials such as, for example, carbohydrates or polyols, to specialty or industrial carboxylic acids and derivatives thereof, and more particularly, e.g., to convert glucose (derived from starch, cellulose or sucrose) to important chemicals such as glucaric acid and derivatives thereof.

### SUMMARY OF THE INVENTION

The present invention is directed to catalyst compositions as defined in the claims.

The catalyst compositions are useful for the selective conversion of a primary hydroxyl group of compositions comprising a primary hydroxyl group and at least a secondary hydroxyl group to a carboxyl group wherein the catalyst compositions are as defined in the claims.

.

The present invention is further directed to processes for preparing glucaric acid or derivatives thereof comprising reacting glucose with a source of oxygen in the presence of a catalyst as defined in the claims and in the substantial absence of added base.

Other objects and features will become apparent and/or will be pointed out hereinafter.

### BRIEF DESCRIPTION OF THE DRAWINGS

Fig. 1 is a TEM photograph of fresh catalyst prepared in accordance with Example 2 (entry 21) comprised of 4 wt% platinum and 4 wt% gold on Saint Gobain Norpro Titania ST31119 particles of 150-250 µm at magnification of 88k.
Fig. 2 is a TEM photograph of fresh catalyst of the present invention as described with respect to Fig. 1 at magnification of 530k.
Fig. 3 is a TEM photograph of fresh catalyst of the present invention as described with respect to Fig. 1 at magnification of 530k.
Fig. 4 is a TEM photograph of fresh catalyst of the present invention as described with respect to Fig. 1 at magnification of 530k.

### DESCRIPTION OF THE PREFERRED EMBODIMENTS

In accordance with the present invention, applicants disclose catalyst compositions as defined in the claims.

The catalyst compositions of the present invention are particularly useful in the selective oxidation of a primary alcohol to a carboxyl in compositions comprising the primary hydroxyl (alcohol) group and at least a secondary hydroxyl (alcohol) group. High conversion and selectivity, coupled with unexpectedly high yield, results from the use of the catalysts of the present invention. Conversion of carbohydrates and, more particularly, glucose and derivatives thereof to oxidation products such as glucaric acid and derivatives thereof is especially efficacious employing the catalysts of the present invention.

The catalyst compositions of the present invention comprise platinum and gold and a support selected from the group consisting of titanias, zirconias, silicas and montmorillonites. These catalysts are heterogeneous, solid-phase catalysts. The metals are present at a surface of the support (i.e., at one or more surfaces, external or internal). The support materials may be modified using methods known in the art such as heat treatment, acid treatment, steam treatment or by the introduction of a dopant (e.g., metal-doped titanias or metal-doped zirconias such as tungstated-zirconia). The catalyst support may be treated so as to promote the preferential deposition of the metals on the outer surface of the support so as to create a shell type catalyst. The supports may be in a variety of form such as powders, pellets, spheres, extrudates and xerogels.

Catalyst performance in general is in significant part dependent upon the degree of uniformity of dispersion of the metals on the support. The platinum and gold-containing catalysts of the present invention may be produced by deposition procedures known in the art including, but not limited to, incipient wetness, ion-exchange and deposition-precipitation. In various embodiments, a uniform dispersion can be effected by forming a heterogeneous slurry of the support in combination with solubilized metal complexes. In certain embodiments, the supports may be initially dispersed in a liquid such as water. Thereafter, in such embodiments, the solubilized metal complexes may be added to the slurry containing the support. The heterogeneous mixture of solid and liquids can then be stirred, mixed and/or shaken to enhance the uniformity of dispersion of the catalyst, which, in turn, enables the more uniform deposition of metals on the surface of the support upon removal of the liquids and additional treatments as may be needed and more fully described hereinafter.

The gold component of the catalysts of the present invention is typically added to the support as a solubilized constituent to enable the formation of a uniform suspension. A base is then added to the suspension in order to create an insoluble gold complex which can be more uniformly deposited onto the support. For example, in various embodiments, the solubilized gold constituent is provided to the slurry as, for example, HAuCl₄. Upon creation of a well dispersed, heterogeneous mixture, a base is added to the slurry to form an insoluble gold complex which then deposits on the surface of the support. Although any base which can effect the formation of an insoluble gold complex is useable, in various embodiments nitrogen-containing bases such as ammonia or urea are employed. It may be desirable, though not required, to collect the support on which has been deposited the insoluble gold complex prior to adding the platinum-containing constituent, which collection can readily be accomplished by any of a variety of means known in the art such as, for example, centrifugation. The collected solids may optionally be washed and then may be heated to dry. Heating may also be employed so as to reduce the gold complex on the support to Au(0). Heating may be conducted at temperatures ranging from about 60°C (to dry) up to about 500°C (at which temperature the gold can be effectively reduced). In various embodiments, the heating step may be conducted in the presence of a reducing or oxidizing atmosphere in order to promote the reduction of the complex to deposit the gold onto the support as Au(0). Heating times vary depending upon, for example, the objective of the heating step and the decomposition rate of the base added to form the insoluble complex, and the heating times can range from a few minutes to a few days. More typically, the heating time for the purpose of drying ranges from about 2 to about 24 hours and for reducing the gold complex is on the order of about 1-4 hours.

In various embodiments, the concentration of the support in the slurry can be in the range of about 1 to about 100 g of solid/liter of slurry, and in other embodiments the concentration can be in the range of about 5 to about 25 g of solid/liter of slurry.

Mixing of the slurry containing the soluble gold-containing compound is continued for a time sufficient to form at least a reasonably uniform suspension. Appropriate times can range from minutes to a few hours. After addition of the base to convert the gold-containing compound to an insoluble gold-containing complex, the uniformity of the slurry should be maintained for a time sufficient to enable the insoluble complex to be formed and deposit on the support. In various embodiments, the time can range from a few minutes to several hours.

The gold deposited on surfaces of the support forms particles comprising gold. According to the invention, the catalyst composition comprises particles of gold-platinum alloy. The gold on the fresh catalyst may exist as particles of Au(0) and/or as gold-containing alloys. The particles size will vary, but the particles are substantially of a size generally less than or equal to about 20 nanometers (nm). By "size" we mean the largest, straight line dimension of the particle, and by "substantially" we mean at least about 50%. More typically, the particles are substantially of a size in the range of about 2 to about 15 nm and, more preferably, in the range of about 5 to about 12 nm.

Platinum is typically added to the support or support-containing slurry after deposition of gold onto the support and, more preferably, after decomposition of the base. Alternatively, the platinum may be added to the support or support-containing slurry prior to the addition of the solubilized gold compound provided the platinum present on the support is in a form that will not be re-dissolved upon the addition of base used to promote the deposition of gold onto the support. The platinum is typically added as a solution of a soluble precursor or as a colloid. Platinum may be added as a compound selected form the group of platinum (II) nitrate, platinum (II) acetylacetonate (acac), tetraamineplatinum (II) nitrate, tetraamineplatinum (II) hydrogenphosphate and tetraamineplatinum (II) hydroxide. More preferred compounds include platinum(II) nitrate, platinum(II) acetylacetonate (acac), and tetraamine platinum(II) nitrate.

Subsequent to the addition of the platinum compound, the support slurry and platinum-containing compound is dried. Drying may be conducted at room temperature or at a temperature up to about 120°C. More preferably, drying is conducted at a temperature in the range of about 40°C to about 80°C and more preferably still at about 60°C. The drying step may be conducted for a period of time ranging from about a few minutes to a few hours. Typically, the drying time is in the range of about 6 hours to about 24 hours.

After drying the support having the platinum compound deposited thereon, it is subjected to at least one thermal treatment in order to reduce platinum deposited as Pt(II) or Pt(IV) to Pt(0). The thermal treatment(s) can be conducted in air or in any reducing or oxidizing atmosphere. In various embodiments the thermal treatment(s) is (are) conducted under a forming gas atmosphere. Alternatively, a liquid reducing agent may be employed to reduce the platinum; for example, hydrazine or formaldehyde may be employed to effect the requisite reduction of the platinum. The atmosphere under which the thermal treatment is conducted is dependent upon the platinum compound employed, with the objective being substantially converting the platinum on the support to Pt(0).

The temperatures at which the thermal treatment(s) is (are) conducted generally range from about 200°C to about 600°C. More typically, the temperatures of the thermal treatment(s) range from about 200°C to about 500°C and, preferably, the range is from about 200°C to bout 400°C. The thermal treatment is typically conducted for a period of time ranging from about 1 hour to about 5 hours. More typically, the treatment is conducted for a period of time ranging from about 1 hour to about 3 hours. For example, at a thermal treatment temperature of about 350°C, the time of the treatment is about 3 hours.

The platinum deposited on surfaces of the support forms particles. Generally, the platinum on the fresh catalyst exists substantially as particles of essentially Pt(0) and/or as platinum-containing alloys such as gold-platinum alloys. In various embodiments, the catalysts will comprise discrete particles of platinum, particles comprising platinum and gold, and, possibly, particles of gold. In certain preferred embodiments, essentially all of the particles consisting essentially of platinum exist as Pt(0). The particles size will vary, but the Pt(0) particles are substantially in the range of from 5 to 30 nanometers. By "size" we mean the largest, straight line dimension of the particle. By "substantially" we mean at least about 50%. Preferably, the particles are at least substantially in the range of about 5 to about 20 nm and, more preferably, in the range of about 8 to about 12 nm.

The platinum: gold molar ratio is from 10:1 to 1:2. More preferably, the platinum:gold molar ratio may vary, for example, from about 3:1 to 1:2. Even more preferably, the molar ratio of platinum:gold is in the range of about 2:1 to about 1:1.

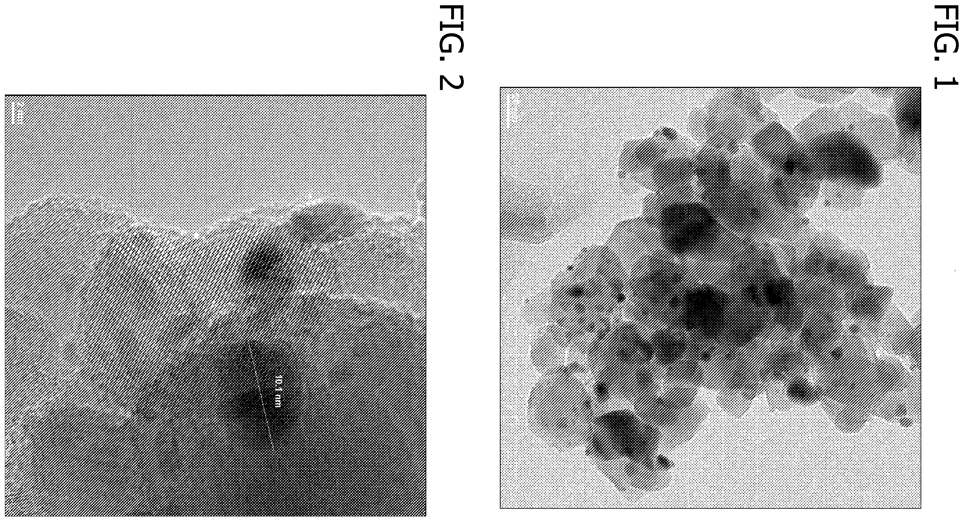

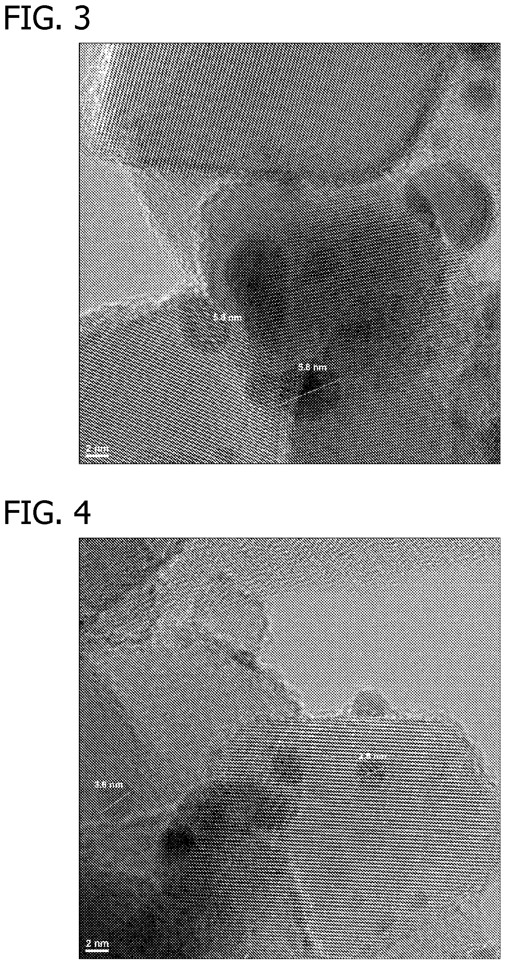

Figure 1 shows a TEM photograph taken at 88k magnification of a 4 wt% Au - 4 wt% Pt catalyst of the present invention prepared in accordance with Example 2 (entry 21) . As shown, metal particles (dark) on the support (light) are less than about 50nm, at least about 50% are less than about 20 nm, and at least about 50% are less than about 15 nm, but generally are equal to or greater than about 2nm. Figures 2, 3 and 4 are photographs of the same fresh catalyst taken at magnifications of 530k showing clearly the presence of metallic particles of about 10 nm and 6 nm, and as small as about 2 nm.

In various other embodiments, a third metal (M3) may be added to produce a Pt/Au/M3 catalyst wherein the M3 metal is not platinum or gold. In yet other embodiments a fourth metal (M4) may be added to produce a Pt/Au/M3/M4 catalyst wherein the M4 metal is not platinum or gold and also not the same metal as the M3 metal. The M3 metal and M4 metal may each be selected from group 6 or group 10 metals, with palladium being a more preferred group 10 metal and tungsten being a more preferred group 6 metal.

The total metal loading on the final catalyst (i.e., excluding any metal that is from the support) is generally less than or equal to about 10 wt % relative to the total catalyst weight. Generally, the total metal loading is in the range of from about 1% to about 10%. More typically, the total weight percent ranges from about 1% to about 8%, and more preferably from about 1% to about 4%.

Glucose is effectively converted to glucaric acid in high yield by reacting glucose with oxygen (as used herein, oxygen can be supplied to the reaction as air, oxygen-enriched air, oxygen alone, or oxygen with other constituents substantially inert to the reaction) in the presence of the catalyst compositions of the present invention and in the absence of added base according to the following reaction:

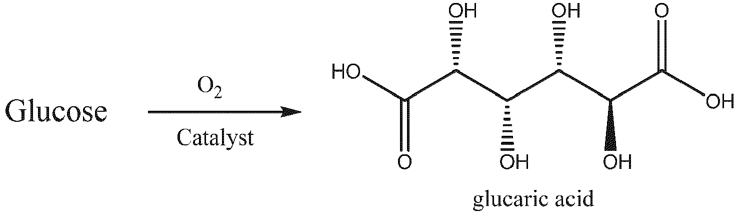

Conducting the oxidation reaction in the absence of added base and in the presence of the catalyst compositions of the present invention does not lead to significant catalyst poisoning effects and oxidation catalyst selectivity is maintained. In fact, catalytic selectivity can be maintained to attain glucaric acid yield in excess of 60%, even 65% and, in some embodiments, attain yields in excess of 70% or higher. The absence of added base advantageously facilitates separation and isolation of the glucaric acid, thereby providing a process that is more amenable to industrial application, and improves overall process economics by eliminating a reaction constituent. The "absence of added base" as used herein means that base, if present (for example, as a constituent of a feedstock), is present in a concentration which has essentially no effect on the efficacy of the reaction (i.e., the oxidation reaction is being conducted essentially free of added base). It has also been discovered that this oxidation reaction can also be conducted in the presence of a weak carboxylic acid, such as acetic acid, in which glucose is soluble. The term "weak carboxylic acid" as used herein means any unsubstituted or substituted carboxylic acid having a pKa of at least about 3.5, more preferably at least about 4.5 and, more particularly, is selected from among unsubstituted acids such as acetic acid, propionic acid or butyric acid, or mixtures thereof.

The initial pH of the reaction mixture is no greater than about 7, and is typically less than 7 such as, for example, 6 or less (e.g., when a weak carboxylic acid is used to solubilize at least a portion of the glucose). The initial pH of the reaction mixture is the pH of the reaction mixture prior to contact with oxygen in the presence of an oxidation catalyst. It is expected that the pH of the reaction mixture after oxygen contact will vary as the reaction proceeds. It is believed that as the concentration of the glucaric acid increases (as the reaction proceeds) the pH will decrease from the initial pH.

The process of producing glucaric acid or derivatives thereof from carbohydrate such as glucose can be conducted with the catalysts of the present invention in the essential absence of nitrogen as an active reaction constituent. Typically, nitrogen is employed in known processes as an oxidant such as in the form of nitrate, in many instances as nitric acid. The use of nitrogen in a form in which it is an active reaction constituent, such as nitrate or nitric acid, results in the need for NOₓ abatement technology and acid regeneration technology, both of which add significant cost to the production of glucaric acid from these known processes, as well as providing a corrosive environment which may deleteriously affect the equipment used to carry out the process. By contrast, for example, in the event air or oxygen-enriched air is used in the oxidation reaction of the present invention as the source of oxygen, the nitrogen is essentially an inactive or inert constituent. Thus, for example, an oxidation reaction employing air or oxygen-enriched air is a reaction conducted essentially free of nitrogen in a form in which it would be an active reaction constituent.

Generally, the temperature of the oxidation reaction mixture is at least about 40°C, more typically 60°C, or higher. In various embodiments, the temperature of the oxidation reaction mixture is from about 40°C to about 150°C, from about 60°C to about 150°C, from about 70°C to about 150°C, from about 70°C to about 140°C, or from about 80°C to about 140°C.

Typically, the partial pressure of oxygen is at least about 15 pounds per square inch absolute (psia) (104 kPa), at least about 25 psia (172 kPa), at least about 40 psia (276 kPa), or at least about 60 psia (414 kPa). In various embodiments, the partial pressure of oxygen is up to about 1000 psia (6895 kPa), or more typically in the range of from about 15 psia (104 kPa) to about 500 psia (3447 kPa).

The oxidation reaction is typically conducted in the presence of a solvent to glucose. Solvents suitable for the oxidation reaction include water and weak carboxylic acids such as acetic acid. Utilization of weak carboxylic acid as a solvent adds cost to the process which cost, as a practical matter, must be balanced against any benefits derived from the use thereof. Thus, suitable solvents for the present invention include water, mixtures of water and weak carboxylic acid, or weak carboxylic acid. The catalyst compositions of the present invention remain stable in the presence of the solvent.

In general, the oxidation reaction can be conducted in a batch, semi-batch, or continuous reactor design using fixed bed reactors, trickle bed reactors, slurry phase reactors, moving bed reactors, or any other design that allows for heterogeneous catalytic reactions. Examples of reactors can be seen in Chemical Process Equipment - Selection and Design, Couper et al., Elsevier 1990. It should be understood that glucose, oxygen, any solvent, and the oxidation catalyst may be introduced into a suitable reactor separately or in various combinations.

The reaction product of the oxidation step will, as described above, contain glucaric acid in considerable and heretofore unexpected fraction, but may also contain derivatives thereof, such as glucarolactones. These glucarolactones, like glucaric acid, constitute a hydrodeoxygenation substrate which is particularly amenable to the production of adipic acid product as hereinafter described. Glucarolactones which may be present in the reaction mixture resulting from the oxidation step include mono and di-lactones such as D-glucaro-1,4-lactone, D-glucaro-6,3- lactone, and D-glucaro-1,4:6,3-dilactone. One advantage of higher concentrations of glucarolactones is further improvement in the economics of a downstream hydrodeoxygenation step for producing adipic acid resulting from a reduction in the amount of water produced.

Glucaric acid produced in accordance with the above may be converted to various other glucaric acid derivatives, such as salts, esters, ketones, and lactones. Methods to convert carboxylic acids to such derivatives are known in the art, see, for example, Wade, Organic Chemistry 3rd ed, Prentice Hall 1995.

Adipic acid is an example of an industrially significant product that can be prepared by chemocatalytic conversion of a glucose source via intermediates such as glucaric acid or derivatives thereof, which intermediates are attainable from the use of the catalysts compositions of the present invention. In this process, a hydrodeoxygenation substrate comprising glucaric acid or derivatives thereof is convertible to an adipic acid product.

The hydrodeoxygenation substrate comprises a compound of the Formula I: 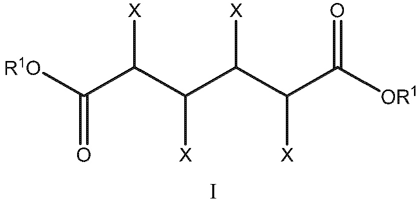 wherein X is independently hydroxyl, oxo, halo, acyloxy or hydrogen provided that at least one X is not hydrogen; R¹ is independently a salt-forming ion, hydrogen, hydrocarbyl, or substituted hydrocarbyl; or a mono- or di-lactone thereof.

As used herein, the term "hydrocarbyl" refers to hydrocarbyl moieties, preferably containing 1 to about 50 carbon atoms, preferably 1 to about 30 carbon atoms, and even more preferably 1 to about 18 carbon atoms, including branched or unbranched, and saturated or unsaturated species. Preferred hydrocarbyl can be selected from the group consisting of alkyl, alkylene, alkoxy, alkylamino, thioalkyl, haloalkyl, cycloalkyl, cycloalkylalkyl, heterocyclyl, N-heterocyclyl, heterocyclylalkyl, aryl, aralkyl heteroaryl, *N-*heteroaryl, heteroarylalkyl, and the like. A hydrocarbyl may be optionally substituted hydrocarbyl. Hence, various hydrocarbyls can be further selected from substituted alkyl, substituted cycloalkyl and the like.

Salt forming ions include, without limitation, for example ammonium ions and metal ions (e.g., alkali and alkaline earth metals). When R¹ is a salt forming ion (i.e., a cation), the carboxyl group may be considered to be anion (i.e., carboxylate anion).

In various embodiments, the hydrodeoxygenation substrate comprises a compound of Formula I, wherein X is hydroxyl and R¹ is independently a salt-forming ion, hydrogen, hydrocarbyl, or substituted hydrocarbyl.

As shown in Formula I, the hydrodeoxygenation substrate contains a six carbon chain comprising four chiral centers. As a result several stereoisomers are possible.

The hydrodeoxygenation substrate may also contain various ketones. For example, not wishing to be bound by theory, when glucaric acid is further oxidized, ketones such as 2-keto-glucaric acid (2,3,4-trihydroxy-5-oxohexanedioic acid) and 3-keto-glucaric acid (2,3,5-trihydroxy-4-oxohexanedioic acid) may be formed.

The hydrodeoxygenation substrate may comprise various lactones derived from glucaric acid. For example, not wishing to be bound by theory, it is believed that various mono- and di-lactones are present in equilibrium with glucaric acid in aqueous solution, including for example, D-glucaro-1,4-lactone, D-glucaro-6,3-lactone, and D-glucaro-1,4:6,3-dilactone. Moreover, processes have been developed to quantitatively convert glucaric acid or a salt thereof in solution to one or more lactones and recover a substantially pure lactone stream. For example see "Convenient Large-Scale Synthesis of D-Glucaro-1,4:6,3-dilactone" Gehret et al., J. Org. Chem., 74 (21), pp. 8373-8376 (2009). Also, lactones such as L-*threo*-4-deoxy-hex-4-enaro-6,3-lactone and L-*erythro*-4-deoxy-hex-4-enaro-6,3-lactone may form from the thermal decomposition of D-Glucaro-1,4:6,3-dilactone.

Therefore, in various embodiments, the hydrodeoxygenation substrate comprises D-glucaro-1,4-lactone. In these and other embodiments, the hydrodeoxygenation substrate comprises D-glucaro-6,3-lactone. Still further, in these and other embodiments, the hydrodeoxygenation substrate comprises D-glucaro-1,4:6,3-dilactone. In these and other embodiments, the hydrodeoxygenation substrate comprises L-*threo*-4-deoxy-hex-4-enaro-6,3-lactone. Still even further, in these and other embodiments, the hydrodeoxygenation substrate comprises L-*erythro*-4-deoxy-hex-4-enaro-6,3-lactone.

When introducing elements of the present invention or the preferred embodiments(s) thereof, the articles "a", "an", "the" and "said" are intended to mean that there are one or more of the elements. The terms "comprising", "including" and "having" are intended to be inclusive and mean that there may be additional elements other than the listed elements.

In view of the above, it will be seen that the several objects of the invention are achieved and other advantageous results attained.

As various changes could be made in the above compositions and processes without departing from the scope of the invention, it is intended that all matter contained in the above description shall be interpreted as illustrative and not in a limiting sense.

Having described the invention in detail, it will be apparent that modifications and variations are possible without departing from the scope of the invention defined in the appended claims.

### EXAMPLES

The following non-limiting examples are provided to further illustrate the present invention.

### Catalyst testing protocol 1

Catalyst (approx. 8 mg) was weighed into a glass vial insert followed by addition of an aqueous glucose solution (250 µl of 10 wt%). The glass vial insert was loaded into a reactor and the reactor was closed. The atmosphere in the reactor was replaced with oxygen and pressurized to 75 psig at room temperature. Reactor was heated to 90°C and maintained at 90°C for 5 hours while vials were shaken. After 5 hours, shaking was stopped and reactor was cooled to 40°C. Pressure in the reactor was then slowly released. The glass vial insert was removed from the reactor and centrifuged. The clear solution was diluted with deionized water and analyzed by ion-chromatography (IC) with conductivity and Corona CAD detection (IC-conductivity-CAD). Samples for IC analysis were prepared by adding to the reaction vial 750 µL water followed by 25-fold dilution. Product yields were determined using a Dionex ICS-3000 Chromatography system equipped with Corona CAD detector (ESA Biosciences) The products were first separated on an Ionpac^{®} AS11-HC column and then quantified by conductivity and Corona CAD detection through comparison with calibration standards.

### Catalyst testing protocol 2

Catalyst (approx. 75 mg) was weighed into a glass vial insert followed by addition of an aqueous glucose solution (2.3 ml of 10 wt%). The glass vial insert was loaded into a reactor and the reactor was closed with a disposable stir paddle fitted to the reaction vessel. The atmosphere in the reactor was replaced with oxygen and pressurized to 50 psig at room temperature. The reactor was heated to a pre-set temperature in the range of 90 to 133°C. After the temperature stabilized, pressure in the reactor was increased to 400 psig by using high pressure oxygen. Stirring was turned on at 1100 rpm. Pressure of 400 psig and the pre-set temperature in the reactor were maintained for an extended period of time using computer control. When a designated reaction time was reached, oxygen feed was stopped, stirring was discontinued and the reactor was cooled to 40°C. Pressure in the reactor was then slowly released. The glass vial insert was removed from the reactor and centrifuged. The clear solution was diluted with deionized water and analyzed by IC-conductivity-CAD as described above.

### Example 1

Approximately 88 µl of an aqueous solution of HAuCl₄ (containing 0.2254 wt% gold) was added to a suspension of 10 mg Titania P25 (Acros Organics) in deionized water (450 µl) while shaking. The suspension was shaken at room temperature for 30 min. 250 µl of an aqueous solution of NH₄OH (4.0 M) was added to the above-mentioned suspension and the resulting suspension was shaken at room temperature for 2 hours. The resulting suspension was then centrifuged and supernatant was decanted. After residual liquid was removed using filter paper, the light yellow solid was dried in a 60°C oven overnight under a dry air purge.

2.1 µl of an aqueous solution of Pt(NO₃)₂ (containing 7.7 wt% platinum) was added to the above solid and the mixture was agitated to impregnate the gold-containing support. The sample was dried in a 60°C oven overnight under a dry air purge. The sample was then reduced at 350°C under forming gas (5% H₂ and 95% N₂) atmosphere for 3 hours with 2°C/min temperature ramp rate. The final catalyst contained about 2.0 wt% gold and 2.0 wt% platinum.

By adjusting the volume and concentration of solutions of HAuCl₄, NH₄OH, and Pt(NO₃)₂, a series of other catalysts with various loadings of gold (0 to 8 wt%) and platinum (0 to 8 wt%) were prepared. Similar methods were also used to prepare catalysts based on other supports, including Titania ST 31119 (Saint-Gobain Norpro), Zirconia Z-1628 (Daiichi Kigenso Kagaku Kogyo), Zirconia Z-1044 (Daiichi Kigenso Kagaku Kogyo), Silica-Titania Cariact (Fuji Silysia), Silica Cariact Q-10 (Fuji Silysia), Davisil 635 (Sigma-Aldrich), and Zeolite CP 811C-300 (Zeolyst). These catalysts were tested for glucose oxidation using Catalyst testing protocol 1 and results are summarized in Table 1.

Entries 3-6, 9, 12, 13, 20, 22, 23, 27-29, 36-39, 44, 45, 48-51, 59, 61, 62, 66-68, 77, 79, 80 and 84 are comparative entries of compositions outside the scope of the present invention. Except for Entry 51, these entries illustrate, when compared to the other entries reported in Table 1, the conversion and other benefits attainable from compositions of the present invention. Entry 51, although exhibiting a higher conversion, causes the production of non-desirable products of over-oxidation at a given glucaric acid yield which render it impractical for commercial use when compared to the catalysts of the present invention. Moreover, many industrial processes employ recycling of unreacted substrate in order to improve overall conversion and a high concentration of non-desirable products reduces the yields ultimately attainable from the feedstock.

**Table 1: Conditions and results of glucose oxidation on gold/platinum catalysts of Example 1**

| Entry | Catalyst wt. (mg) | Support | Au wt.% | Pt wt.% | Glucose Conversion (%) | Glucaric Acid Yield (%) |
|---|---|---|---|---|---|---|
| 1 | 8.3 | Silica Cariact Q-10 | 0.8 | 7.2 | 98 | 51 |
| 2 | 8.0 | Silica Cariact Q-10 | 2.8 | 1.2 | 100 | 36 |
| 3 | 8.1 | Silica Cariact Q-10 | 3.2 | 0.8 | 100 | 28 |
| 4 | 7.7 | Silica Cariact Q-10 | 4.0 | 0.0 | 99 | 4 |
| 5 | 8.4 | Silica Cariact Q-10 | 8.0 | 0.0 | 100 | 6 |
| 6 | 8.3 | Silica Davisil 635 | 0.0 | 8.0 | 96 | 44 |
| 7 | 8.2 | Silica Davisil 635 | 1.6 | 6.4 | 100 | 53 |
| 8 | 8.4 | Silica Davisil 635 | 2.4 | 1.6 | 100 | 46 |
| 9 | 8.5 | Silica Davisil 635 | 8.0 | 0.0 | 100 | 6 |
| 10 | 8.5 | Silica-Titania Cariact | 2.8 | 1.2 | 100 | 29 |
| 11 | 8.5 | Silica-Titania Cariact | 5.6 | 2.4 | 100 | 40 |
| 12 | 8.5 | Silica-Titania Cariact | 7.2 | 0.8 | 100 | 20 |
| 13 | 8.2 | Titania P25 | 0.0 | 8.0 | 86 | 33 |
| 14 | 8.4 | Titania P25 | 0.4 | 3.6 | 97 | 33 |
| 15 | 8.2 | Titania P25 | 1.2 | 2.8 | 100 | 39 |
| 16 | 7.8 | Titania P25 | 1.6 | 2.4 | 100 | 45 |
| 17 | 8.4 | Titania P25 | 1.6 | 6.4 | 100 | 62 |
| 18 | 8.3 | Titania P25 | 2.0 | 2.0 | 100 | 33 |
| 19 | 8.5 | Titania P25 | 2.4 | 5.6 | 100 | 64 |
| 20 | 8.3 | Titania P25 | 3.2 | 0.8 | 100 | 11 |
| 21 | 7.7 | Titania P25 | 3.2 | 4.8 | 100 | 57 |
| 22 | 8.3 | Titania P25 | 3.6 | 0.4 | 100 | 5 |
| 23 | 8.0 | Titania P25 | 4.0 | 0.0 | 100 | 1 |
| 24 | 7.8 | Titania P25 | 4.0 | 4.0 | 100 | 46 |
| 25 | 7.8 | Titania P25 | 4.8 | 32 | 100 | 21 |
| 26 | 8.0 | Titania P25 | 5.6 | 2.4 | 100 | 18 |
| 27 | 8.2 | Titania P25 | 6.4 | 1.6 | 100 | 14 |
| 28 | 7.6 | Titania P25 | 7.2 | 0.8 | 100 | 8 |
| 29 | 7.8 | Titania P25 | 8.0 | 0.0 | 100 | 1 |
| 30 | 8.1 | Titania ST 31119 | 0.8 | 3.2 | 100 | 40 |
| 31 | 8.5 | Titania ST 31119 | 1.2 | 2.8 | 100 | 49 |
| 32 | 7.7 | Titania ST 31119 | 1.6 | 2.4 | 100 | 42 |
| 33 | 7.9 | Titania ST 31119 | 2.0 | 2.0 | 100 | 43 |
| 34 | 8.0 | Titania ST 31119 | 2.4 | 1.6 | 100 | 25 |
| 35 | 7.6 | Titania ST 31119 | 2.8 | 1.2 | 100 | 22 |
| 36 | 8.5 | Titania ST 31119 | 3.2 | 0.8 | 100 | 21 |
| 37 | 7.9 | Titania ST 31119 | 3.6 | 0.4 | 100 | 13 |
| 38 | 8.1 | Titania ST 31119 | 4.0 | 0.0 | 100 | 2 |
| 39 | 8.4 | Titania ST 31119 | 8.0 | 0.0 | 96 | 1 |
| 40 | 8.0 | Zeolite CP 811C-300 | 0.8 | 3.2 | 93 | 22 |
| 41 | 8.1 | Zeolite CP 811C-300 | 12 | 2.8 | 100 | 29 |
| 42 | 8.2 | Zeolite CP 811C-300 | 2.4 | 1.6 | 100 | 28 |
| 43 | 7.9 | Zeolite CP 811C-300 | 2.8 | 1.2 | 100 | 20 |
| 44 | 7.7 | Zeolite CP 811C-300 | 3.2 | 0.8 | 100 | 14 |
| 45 | 8.3 | Zeolite CP 811C-300 | 3.6 | 0.4 | 99 | 6 |
| 46 | 8.0 | Zeolite CP 811C-300 | 4.8 | 3.2 | 100 | 41 |
| 47 | 8.4 | Zeolite CP 811C-300 | 5.6 | 2.4 | 100 | 31 |
| 48 | 7.7 | Zeolite CP 811C-300 | 6.4 | 1.6 | 100 | 20 |
| 49 | 7.9 | Zeolite CP 811C-300 | 8.0 | 0.0 | 100 | 7 |
| 50 | 7.9 | Zirconia 1628 | 0.0 | 4.0 | 84 | 23 |
| 51 | 8.2 | Zirconia 1628 | 0.0 | 8.0 | 98 | 50 |
| 52 | 8.1 | Zirconia 1628 | 0.4 | 3.6 | 85 | 22 |
| 53 | 8.3 | Zirconia 1628 | 0.8 | 3.2 | 97 | 22 |
| 54 | 8.3 | Zirconia 1628 | 0.8 | 7.2 | 98 | 45 |
| 55 | 8.0 | Zirconia 1628 | 1.2 | 2.8 | 100 | 25 |
| 56 | 7.8 | Zirconia 1628 | 1.6 | 2.4 | 100 | 22 |
| 57 | 8.2 | Zirconia 1628 | 2.4 | 1.6 | 100 | 16 |
| 58 | 7.9 | Zirconia 1628 | 2.8 | 1.2 | 100 | 17 |
| 59 | 8.4 | Zirconia 1628 | 3.2 | 0.8 | 100 | 14 |
| 60 | 8.5 | Zirconia 1628 | 3.2 | 4.8 | 100 | 40 |
| 61 | 7.8 | Zirconia 1628 | 3.6 | 0.4 | 100 | 7 |
| 62 | 7.8 | Zirconia 1628 | 4.0 | 0.0 | 99 | 0 |
| 63 | 7.9 | Zirconia 1628 | 4.0 | 4.0 | 100 | 33 |
| 64 | 8.3 | Zirconia 1628 | 4.8 | 3.2 | 100 | 24 |
| 65 | 8.0 | Zirconia 1628 | 5.6 | 2.4 | 100 | 16 |
| 66 | 8.0 | Zirconia 1628 | 6.4 | 1.6 | 100 | 9 |
| 67 | 8.2 | Zirconia 1628 | 8.0 | 0.0 | 71 | 0 |
| 68 | 7.6 | Zirconia Z-1044 | 0.0 | 4.0 | 72 | 19 |
| 69 | 7.9 | Zirconia Z-1044 | 0.4 | 3.6 | 88 | 29 |
| 70 | 7.8 | Zirconia Z-1044 | 0.8 | 3.2 | 100 | 40 |
| 71 | 8.1 | Zirconia Z-1044 | 1.2 | 2.8 | 100 | 44 |
| 72 | 7.8 | Zirconia Z-1044 | 1.6 | 2.4 | 100 | 36 |
| 73 | 8.2 | Zirconia Z-1044 | 2.0 | 2.0 | 100 | 36 |
| 74 | 8.0 | Zirconia Z-1044 | 2.4 | 1.6 | 100 | 32 |
| 75 | 7.5 | Zirconia Z-1044 | 2.4 | 5.6 | 100 | 58 |
| 76 | 8.1 | Zirconia Z-1044 | 2.8 | 1.2 | 100 | 26 |
| 77 | 7.9 | Zirconia Z-1044 | 3.2 | 0.8 | 100 | 19 |
| 78 | 7.9 | Zirconia Z-1044 | 3.2 | 4.8 | 100 | 58 |
| 79 | 8.0 | Zirconia Z-1044 | 3.6 | 0.4 | 100 | 7 |
| 80 | 8.5 | Zirconia Z-1044 | 4.0 | 0.0 | 100 | 3 |
| 81 | 8.0 | Zirconia Z-1044 | 4.0 | 4.0 | 100 | 57 |
| 82 | 8.0 | Zirconia Z-1044 | 4.8 | 3.2 | 100 | 47 |
| 83 | 7.6 | Zirconia Z-1044 | 5.6 | 2.4 | 100 | 31 |
| 84 | 7.7 | Zirconia Z-1044 | 6.4 | 1.6 | 100 | 16 |

### Example 2

88 µl of an aqueous solution of HAuCl₄ (containing 0.2254 wt% gold) was added to a suspension of 10 mg titania (P25, Acros Organics) in deionized water (550 µl) while shaking. The suspension was shaken at room temperature for 30 min. 250 µl of an aqueous solution of NH₄OH (4.0 M) was added to above suspension and the resulting suspension was shaken at room temperature for 2 hours. The suspension was then centrifuged and supernatant was decanted. After residual liquid was removed using filter paper, the light yellow solid was dried in a 60°C oven overnight under a dry air purge.

Another batch of material was prepared by repeating above preparation with exception that after the light yellow solid was collected, it was washed with deionized water (2 times of 500 µl each) before it was dried in a 60°C oven overnight under a dry air purge.

2.1 µl of an aqueous solution of Pt(NO₃)₂ (containing 7.7 wt% platinum) was added to the above solids and the mixtures were agitated to impregnate the gold-containing supports. The samples were dried in a 60°C oven overnight under a dry air purge. The samples were then reduced at 350°C under forming gas (5% H₂ and 95% N₂) atmosphere for 3 hours with 2°C/min temperature ramp rate. The final catalysts contained approximately 2.0 wt% gold and 2.0 wt% platinum.

By adjusting the volume and concentration of solutions of HAuCl₄, NH₄OH, and Pt(NO₃)₂, a series of other catalysts with various loadings of gold (2 or 4 wt%) and platinum (2 or 4 wt%) were prepared using the Zirconia Z-1628 (Daiichi Kigenso Kagaku Kogyo), Silica-Titania Cariact (Fuji Silysia) and Silica Cariact Q-10 (Fuji Silysia).

These catalysts were tested for glucose oxidation using Catalyst testing protocol 1 and results are summarized in Table 2.

**Table 2: Conditions and results of glucose oxidation on gold/platinum catalysts of Example 2**

| Entry | Catalyst Amount (mg) | Support | Au wt% | Pt wt% | Wash after Au deposition | Glucose Conversion (%) | Glucaric Acid Yield (%) |
|---|---|---|---|---|---|---|---|
| 4 | 7.0 | Silica Cariact Q-10 | 2 | 2 | No | 100 | 28 |
| 5 | 7.2 | Silica Cariact Q-10 | 2 | 2 | Yes | 100 | 28 |
| 7 | 7.1 | Silica Cariact Q-10 | 4 | 4 | Yes | 100 | 48 |
| 8 | 8.0 | Silica Cariact Q-10 | 4 | 4 | No | 100 | 44 |
| 21 | 7.1 | Titania ST 31119 | 4 | 4 | No | 100 | 62 |
| 23 | 7.4 | Titania-Silica Cariact | 4 | 4 | Yes | 100 | 39 |
| 24 | 7.4 | Titania-Silica Cariact | 4 | 4 | No | 100 | 41 |
| 29 | 6.6 | Zirconia 1628 | 2 | 2 | Yes | 100 | 26 |
| 30 | 6.6 | Zirconia 1628 | 2 | 2 | No | 100 | 22 |

### Example 3

88 µl of an aqueous solution of HAuCl₄ (containing 0.2254 wt% gold) was added to a suspension of 10 mg titania (P25, Acros Organics) in deionized water (1900 µl) while shaking. The suspension (at concentration of 5 mg support/ml) was shaken at room temperature for 30 min. 400 µl of an aqueous solution of urea (20 wt%) was added to above suspension and the resulting suspension was heated at 80°C for 2 hours while shaking. The suspension was then cooled to room temperature, centrifuged and supernatant was decanted. After residual liquid was removed using filter paper, the light yellow solid was dried in a 60°C oven overnight under a dry air purge.

Another batch of material was prepared by repeating above preparation with exception that after the light yellow solid was collected, it was washed with deionized water (2 times of 2000 µl each) before it was dried in a 60°C oven overnight under a dry air purge.

2.1 µl of an aqueous solution of Pt(NO₃)₂ (containing 7.7 wt% platinum) was added to above solid and the mixture was agitated to impregnate the gold-containing support. The sample was dried in a 60°C oven overnight under a dry air purge. The sample was then reduced at 350°C under forming gas (5% H₂ and 95% N₂) atmosphere for 3 hours with 2°C/min temperature ramp rate. The final catalyst contained approximately 2.0 wt% gold and 2.0 wt% platinum.

Similar method was also used to prepare catalysts based on other supports, including Zirconia Z-1628 (Daiichi Kigenso Kagaku Kogyo), Zirconia Z-1044 (Daiichi Kigenso Kagaku Kogyo), Silica-Titania Cariact (Fuji Silysia) and Silica Cariact Q-10 (Fuji Silysia).

These catalysts were tested for glucose oxidation using Catalyst testing protocol 1 and results are summarized in Table 3.

**Table 3: Conditions and results of glucose oxidation on gold/platinum catalysts of Example 3**

| Entry | Support | Wash after Au deposition | Concentration of Suspension (mg support/ml) | Catalyst Amount (mg) | Glucose Conversion (%) | Glucaric Acid Yield (%) |
|---|---|---|---|---|---|---|
| 1 | Silica Cariact Q-10 | No | 5 | 9.2 | 100 | 23 |
| 2 | Silica Cariact Q-10 | Yes | 5 | 7.0 | 100 | 17 |
| 3 | Silica-Titania | No | 5 | 8.8 | 100 | 30 |
| 4 | Silica-Titania | Yes | 5 | 8.4 | 100 | 34 |
| 5 | Titania P25 | No | 5 | 7.4 | 100 | 35 |
| 6 | Titania P25 | Yes | 5 | 7.0 | 100 | 37 |
| 7 | Zirconia 1628 | No | 5 | 7.4 | 80 | 11 |
| 8 | Zirconia 1628 | Yes | 5 | 7.7 | 100 | 22 |
| 9 | Zirconia Z-1044 | No | 5 | 7.8 | 100 | 22 |
| 10 | Zirconia Z-1044 | Yes | 5 | 7.2 | 100 | 26 |

### Example 4

12 µl of an aqueous solution of HAuCl₄ (containing 22.54 wt% gold) was added to a suspension of 187 mg Silica Cariact Q-10 (Fuji Silysia) in deionized water (38 ml) while stirring. The suspension was stirred at room temperature for 30 min. 100 µl of an aqueous solution of NH₄OH (15.85 M) was added to above suspension and the resulting suspension was stirred at room temperature for 2 hours. The suspension was then centrifuged and supernatant was decanted. The light yellow solid was washed with deionized water (2 times of 35 ml each) at 50°C before it was dried in a 60°C oven overnight under a dry air purge. The sample was then reduced at 200°C under forming gas (5% H₂ and 95% N₂) atmosphere for 3 hours with 2°C/min temperature ramp rate.

10 mg of dry material from above preparation was used for the following synthesis.

12.8 µl of an aqueous solution of Pt(NO₃)₂ (containing 1.5 wt% platinum) was added to above solid (10 mg) and the mixture was agitated to impregnate the gold-containing support. The sample was dried in a 60°C oven overnight under a dry air purge. The sample was then reduced at 350°C under forming gas (5% H₂ and 95% N₂) atmosphere for 3 hours with 2°C/min temperature ramp rate. The final catalyst contained approximately 2.0 wt% gold and 2.0 wt% platinum.

The catalyst was tested for glucose oxidation using Catalyst testing protocol 1 and results are summarized in Table 4.

**Table 4: Conditions and results of glucose oxidation on gold/platinum catalysts of Example 4**

| Entry | Support | Catalyst Amount (mg) | Glucose Conversion (%) | Glucaric Acid Yield (%) |
|---|---|---|---|---|
| 1 | Silica Cariact Q-10 | 8.3 | 100 | 48 |

### Example 5

12 µl of an aqueous solution of HAuCl₄ (containing 22.54 wt% gold) was added to a suspension of 187 mg Silica Cariact Q-10 (Fuji Silysia) in deionized water (38 ml) while stirring. The suspension was stirred at room temperature for 30 min. 100 µl of an aqueous solution of NH₄OH (15.85 M) was added to above suspension and the resulting suspension was stirred at room temperature for 2 hours. The suspension was then centrifuged and supernatant was decanted. The light yellow solid was washed with deionized water (2 times of 35 ml each) at 50°C before it was dried in a 60°C oven overnight under a dry air purge. The sample was then reduced at 200°C under forming gas (5% H₂ and 95% N₂) atmosphere for 3 hours with 2°C/min temperature ramp rate.

10 mg of dry material from above preparation was used for the following synthesis.

12.8 µl of an aqueous solution of Pt(NO₃)₂ (containing 1.5 wt% platinum) was added to above solid (10 mg) and the mixture was agitated to impregnate the gold-containing support. The sample was dried in a 60°C oven overnight under a dry air purge. The sample was calcined at 350°C in air for 3 hours with 5°C/min temperature ramp rate, then reduced at 350°C under forming gas (5% H₂ and 95% N₂) atmosphere for 3 hours with 2°C/min temperature ramp rate. The final catalyst contained approximately 2.0 wt% gold and 2.0 wt% platinum.

The catalyst was tested for glucose oxidation using Catalyst testing protocol 1 and results are summarized in Table 5.

**Table 5: Conditions and results of glucose oxidation on gold/platinum catalysts of Example 5**

| Entry | Support | Catalyst Amount (mg) | Glucose Conversion (%) | Glucaric Acid Yield (%) |
|---|---|---|---|---|
| 1 | Silica Cariact Q-10 | 8.7 | 100 | 43 |

### Example 6

12 µl of an aqueous solution of HAuCl₄ (containing 22.54 wt% gold) was added to a suspension of 187 mg Silica Cariact Q-10 (Fuji Silysia) in deionized water (38 ml) while stirring. The suspension was stirred at room temperature for 30 min. 100 µl of an aqueous solution of NH₄OH (15.85 M) was added to above suspension and the resulting suspension was stirred at room temperature for 2 hours. The suspension was then centrifuged and supernatant was decanted. The light yellow solid was washed with deionized water (2 times of 35 ml each) at 50°C before it was dried in a 60°C oven overnight under a dry air purge. The sample was then calcined at 350°C in air for 3 hours with 5°C/min temperature ramp rate.

10 mg of dry material from above preparation was used for the following synthesis.

12.8 µl of an aqueous solution of Pt(NO₃)₂ (containing 1.5 wt% platinum) was added to above solid (10 mg) and the mixture was agitated to impregnate the gold-containing support. The sample was dried in a 60°C oven overnight under a dry air purge. The sample was then reduced at 350°C under forming gas (5% H₂ and 95% N₂) atmosphere for 3 hours with 2°C/min temperature ramp rate. The final catalyst contained approximately 2.0 wt% gold and 2.0 wt% platinum.

The catalyst was tested for glucose oxidation using Catalyst testing protocol 1 and results are summarized in Table 6.

**Table 6: Conditions and results of glucose oxidation on gold/platinum catalysts of Example 6**

| Entry | Support | Catalyst Amount (mg) | Glucose Conversion (%) | Glucaric Acid Yield (%) |
|---|---|---|---|---|
| 1 | Silica Cariact Q-10 | 8.4 | 100 | 42 |

### Example 7

625 µl of an aqueous solution of HAuCla (containing 22.54 wt% gold) was added to a suspension of 5.0 g titania (P25, Acros Organics) in deionized water (500 ml) while stirring. The suspension was stirred at room temperature for 30 min. 30 ml of an aqueous solution of urea (20 wt%) was added to above suspension and the resulting suspension was heated at 80°C for overnight while stirring. The suspension was then cooled to room temperature, centrifuged and supernatant was decanted. The light yellow solid was washed with deionized water (3 times of 400 ml each) at 50°C before it was dried in a 60°C oven overnight under a dry air purge. The sample was then reduced at 200°C under forming gas (5% H₂ and 95% N₂) atmosphere for 3 hours with 2°C/min temperature ramp rate to give 4.90 g purple solid.

1.225 ml of an aqueous solution of Pt(NO₃)₂ (containing 11.4 wt% platinum) was added to the above purple solid in 4 portions. After each addition, the mixture was agitated to impregnate the gold-containing support. The sample was dried in a 60°C oven overnight under a dry air purge. The sample was then reduced at 350°C under forming gas (5% H₂ and 95% N₂) atmosphere for 3 hours with 2°C/min temperature ramp rate. The final catalyst contained approximately 4.0 wt% gold and 4.0 wt% platinum.

The catalyst was tested for glucose oxidation using Catalyst testing protocol 2 and results are summarized in Table 7.

**Table 7: Conditions and results of glucose oxidation on gold/platinum catalyst of Example 7**

| Entry | Temperature (°C) | Reaction Time (h) | Glucose Conversion (%) | Glucaric Acid Yield (%) |
|---|---|---|---|---|
| 1 | 91 | 3.0 | 100 | 42 |
| 2 | 91 | 5.0 | 100 | 50 |
| 3 | 98 | 3.0 | 100 | 49 |
| 4 | 98 | 5.0 | 100 | 59 |
| 5 | 105 | 3.0 | 100 | 59 |
| 6 | 105 | 5.0 | 100 | 67 |
| 7 | 112 | 3.0 | 100 | 67 |
| 8 | 112 | 5.0 | 100 | 70 |
| 9 | 119 | 2.0 | 100 | 66 |
| 10 | 133 | 1.0 | 100 | 65 |

### Example 8

Method similar to that in Example 7 was used to prepare catalyst based on titania (ST 31119, Saint-Gobain Norpro).

This catalyst was tested for glucose oxidation using Catalyst testing protocol 2 and results are summarized in Table 8.

**Table 8: Conditions and results of glucose oxidation on gold/platinum catalyst of Example 8**

| Entry | Temperature (°C) | Reaction Time (h) | Glucose Conversion (%) | Glucaric Acid Yield (%) |
|---|---|---|---|---|
| 1 | 91 | 5.0 | 100 | 51 |
| 2 | 98 | 3.0 | 100 | 52 |
| 3 | 98 | 5.0 | 100 | 59 |
| 4 | 105 | 3.0 | 100 | 58 |
| 5 | 112 | 3.0 | 100 | 68 |
| 6 | 119 | 2.0 | 100 | 69 |
| 7 | 119 | 3.0 | 100 | 71 |
| 8 | 126 | 2.0 | 100 | 70 |

### Example 9

312 µl of an aqueous solution of HAuCh (containing 22.54 wt% gold) was added to a suspension of 5.0 g titania (P25, Acros Organics) in deionized water (500 ml) while stirring. The suspension was stirred at room temperature for 30 min. 15 ml of an aqueous solution of urea (20 wt%) was added to above suspension and the resulting suspension was heated at 80°C for overnight while stirring. The suspension was then cooled to room temperature, centrifuged and supernatant was decanted. The light yellow solid was washed with deionized water (3 times of 400 ml each) at 50°C before it was dried in a 60°C oven overnight under a dry air purge. The sample was then reduced at 200°C under forming gas (5% H₂ and 95% N₂) atmosphere for 3 hours with 2°C/min temperature ramp rate to give 4.90 g purple solid.

612 µl of an aqueous solution of Pt(NO₃)₂ (containing 11.4 wt% platinum) was added to above purple solid in 4 portions. After each addition, the mixture was agitated to impregnate the gold-containing support. The sample was dried in a 60°C oven overnight under a dry air purge. The sample was then reduced at 350°C under forming gas (5% H₂ and 95% N₂) atmosphere for 3 hours with 2°C/min temperature ramp rate. The final catalyst contained approximately 2.0 wt% gold and 2.0 wt% platinum.

This catalyst was tested for glucose oxidation using Catalyst testing protocol 2 and results are summarized in Table 9.

**Table 9: Conditions and results of glucose oxidation on gold/platinum catalyst of Example 9**

| Entry | Temperature (°C) | Reaction time (h) | Substrate Glucose wt% | Glucose Conversion (%) | Glucaric Acid Yield (%) |
|---|---|---|---|---|---|
| 1 | 90 | 5.0 | 10 | 100 | 32 |
| 2 | 90 | 3.0 | 10 | 100 | 26 |
| 3 | 90 | 5.0 | 20 | 100 | 21 |

### Example 10

88 µl of an aqueous solution of HAuCl₄ (containing 0.2254 wt% gold) was added to a suspension of 10 mg titania (ST 31119, Saint-Gobain Norpro) in deionized water (450 µl) while shaking. The suspension was shaken at room temperature for 30 min. 250 µl of an aqueous solution of NH₄OH (4.0 M) was added to above suspension and the resulting suspension was shaken at room temperature for 2 hours. The suspension was then centrifuged and supernatant was decanted. After residual liquid was removed using filter paper, the light yellow solid was dried in a 60°C oven overnight under a dry air purge.

2.1 µl of an aqueous solution of (NH₃)₄Pt(NO₃)₂ (containing 7.7 wt% platinum) was added to above solid and the mixture was agitated to impregnate the gold-containing support. The sample was dried in a 60°C oven overnight under a dry air purge. The sample was then reduced at 350°C under forming gas (5% H₂ and 95% N₂) atmosphere for 3 hours with 2°C/min temperature ramp rate. The final catalyst contained approximately 2.0 wt% gold and 2.0 wt% platinum.

By adjusting the volume and concentration of solutions of HAuCl₄, NH₄OH, and (NH₃)₄Pt(NO₃)₂, a series of other catalysts with various loadings of gold (0 to 2 wt%) and platinum (0 to 2 wt%) were prepared. Similar method was also used to prepare catalysts based on Zirconia Z-1044 (Daiichi Kigenso Kagaku Kogyo). These catalysts were tested for glucose oxidation using Catalyst testing protocol 1 and results are summarized in Table 10.

**Table 10: Conditions and results of glucose oxidation on gold/platinum catalyst of Example 10**

| Entry | Catalyst Amount (mg) | Support | Au wt% | Pt wt% | Glucose Conversion (%) | Glucaric Acid Yield (%) |
|---|---|---|---|---|---|---|
| 1 | 8.8 | Titania ST 31119 | 1.4 | 2.6 | 100 | 28 |
| 2 | 8.6 | Titania ST 31119 | 2.0 | 2.0 | 100 | 30 |
| 3 | 9 | Zirconia Z-1044 | 1.4 | 2.6 | 100 | 35 |
| 4 | 8.6 | Zirconia Z-1044 | 2.0 | 2.0 | 100 | 17 |

### Example 11

2.1 µl of an aqueous solution of Pt(NO₃)₂ (containing 7.7 wt% platinum) was added to 10 mg titania (ST 31119, Saint-Gobain Norpro) and the mixture was agitated to impregnate the support. The sample was dried in a 60°C oven overnight under a dry air purge. The sample was then reduced at 350°C under forming gas (5% H₂ and 95% N₂) atmosphere for 3 hours with 2°C/min temperature ramp rate.

88 µl of an aqueous solution of HAuCl₄ (containing 0.2254 wt% gold) was added to a suspension of above solid in deionized water (550 µl) while shaking. The suspension was shaken at room temperature for 30 min. 250 µl of an aqueous solution of NH₄OH (4.0 M) was added to above suspension and the resulting suspension was shaken at room temperature for 2 hours. The suspension was then centrifuged and supernatant was decanted. After residual liquid was removed using filter paper, the solid was collected and dried in a 60°C oven overnight under a dry air purge. The sample was then reduced at 350°C under forming gas (5% H₂ and 95% N₂) atmosphere for 3 hours with 2°C/min temperature ramp rate. The final catalyst contained approximately 2.0 wt% gold and 2.0 wt% platinum.

By adjusting the volume and concentration of solutions of HAuCl₄, NH₄OH, and Pt(NO₃)₂, a series of other catalysts with various loadings of gold (0.4 to 2 wt%) and platinum (1.0 to 3.2 wt%) were prepared. Similar method was also used to prepare catalysts based on Zirconia Z-1044 (Daiichi Kigenso Kagaku Kogyo). These catalysts were tested for glucose oxidation using Catalyst testing protocol 1 and results are summarized in Table 11.

**Table 11: Conditions and results of glucose oxidation on gold/platinum catalyst of Example 11**

| Entry | Catalyst Amount (mg) | Support | Au wt% | Pt wt% | Glucose Conversion (%) | Glucaric Acid Yield (%) |
|---|---|---|---|---|---|---|
| 1 | 8.2 | Titania ST 31119 | 0.4 | 1.6 | 72 | 15 |
| 2 | 8.7 | Titania ST 31119 | 0.7 | 1.3 | 100 | 25 |
| 3 | 8.2 | Titania ST 31119 | 0.8 | 3.2 | 93 | 30 |
| 4 | 7.9 | Titania ST 31119 | 1.0 | 1.0 | 100 | 21 |
| 5 | 8.9 | Titania ST 31119 | 1.4 | 2.6 | 100 | 43 |
| 6 | 8.8 | Titania ST 31119 | 2.0 | 2.0 | 100 | 43 |
| 7 | 7.9 | Zirconia Z-1044 | 0.4 | 1.6 | 72 | 11 |
| 8 | 7.9 | Zirconia Z-1044 | 0.7 | 1.3 | 100 | 27 |
| 9 | 8.4 | Zirconia Z-1044 | 0.8 | 3.2 | 97 | 33 |
| 10 | 7.3 | Zirconia Z-1044 | 1.0 | 1.0 | 100 | 27 |
| 11 | 8 | Zirconia Z-1044 | 1.4 | 2.6 | 100 | 47 |
| 12 | 8.3 | Zirconia Z-1044 | 2.0 | 2.0 | 100 | 42 |

### Example 12

2.1 µl of an aqueous solution of Pt(NO₃)₂ (containing 7.7 wt% platinum) was added to 10 mg titania (ST 31119, Saint-Gobain Norpro) and the mixture was agitated to impregnate the support. The sample was dried in a 60°C oven overnight under a dry air purge. The sample was then calcined at 500°C in air for 3 hours with 5°C/min temperature ramp rate.

88 µl of an aqueous solution of HAuCl₄ (containing 0.2254 wt% gold) was added to a suspension of above solid in deionized water (550 µl) while shaking. The suspension was shaken at room temperature for 30 min. 250 µl of an aqueous solution of NH₄OH (4.0 M) was added to above suspension and the resulting suspension was shaken at room temperature for 2 hours. The suspension was then centrifuged and supernatant was decanted. After residual liquid was removed using filter paper, the solid was dried in a 60°C oven overnight under a dry air purge. The sample was then reduced at 350°C under forming gas (5% H₂ and 95% N₂) atmosphere for 3 hours with 2°C/min temperature ramp rate. The final catalyst contained approximately 2.0 wt% gold and 2.0 wt% platinum.

By adjusting the volume and concentration of solutions of HAuCl₄, NH₄OH, and Pt(NO₃)₂, a series of other catalysts with various loadings of gold (0 to 2 wt%) and platinum (0 to 2 wt%) were prepared. Similar method was also used to prepare catalysts based on Zirconia Z-1044 (Daiichi Kigenso Kagaku Kogyo). These catalysts were tested for glucose oxidation using Catalyst testing protocol 1 and results are summarized in Table 12.

**Table 12: Conditions and results of glucose oxidation on gold/platinum catalyst of Example 12**

| Entry | Catalyst Amount (mg) | Support | Au wt% | Pt wt% | Glucose Conversion (%) | Glucaric Acid Yield (%) |
|---|---|---|---|---|---|---|
| 1 | 7.9 | Titania ST 31119 | 0.4 | 1.6 | 85 | 17 |
| 2 | 8.1 | Titania ST 31119 | 0.7 | 1.3 | 100 | 28 |
| 3 | 7.9 | Titania ST 31119 | 0.8 | 3.2 | 100 | 44 |
| 4 | 8.2 | Titania ST 31119 | 1.0 | 1.0 | 100 | 21 |
| 5 | 7.7 | Titania ST 31119 | 1.4 | 2.6 | 100 | 49 |
| 6 | 8.2 | Titania ST 31119 | 2.0 | 2.0 | 100 | 43 |
| 7 | 7.8 | Zirconia Z-1044 | 0.4 | 1.6 | 99 | 23 |
| 8 | 8.2 | Zirconia Z-1044 | 0.7 | 1.3 | 100 | 30 |
| 9 | 7.7 | Zirconia Z-1044 | 0.8 | 3.2 | 100 | 46 |
| 10 | 7.9 | Zirconia Z-1044 | 1.0 | 1.0 | 100 | 27 |
| 11 | 9.2 | Zirconia Z-1044 | 1.4 | 2.6 | 100 | 51 |
| 12 | 8.5 | Zirconia Z-1044 | 2.0 | 2.0 | 100 | 40 |

### Example 13

### Preparation of platinum/gold catalyst from Au/TiO2 [Sud Chemie 02-10]

Pt(NO₃)₂ solution was added to a dried sample of the commercial 1.5% gold/titania catalyst [Süd Chemie 02-10] (wherein the total volume of the Pt(NO₃)₂ volume was matched to equal to the pore volume of the catalyst) with agitation, whereupon the material was dried in a furnace at 120°C for 1 hour, followed by reduction under flowing 5 vol. % H₂ in N₂ at 350°C for 3 hours. The results are presented as Entry 1 of Table 13.

### Preparation of 4 wt% platinum/4 wt% gold catalyst

Pt(NO₃)₂ solution and AuCl₂ solution was added to a dried sample of Titania NorPro ST 61120 (Saint Gobain Norpro) with agitation, whereupon the material was dried in a furnace at 120°C for 16 hours. A Pt(NO₃)₂ solution was subsequently added to the dried sample of Au / Titania NorPro ST 61120 with agitation, whereupon the material was dried in a furnace at 120°C for 16 hours followed by a reduction at 350°C under flowing 5 vol.% H₂ in N₂ for 3 hours. The results are presented as Entry 2 of Table 13.

**Table 13**

| Entry | Catalyst (wt. % M2 wt.% Pt/Support) | M1 Precursor | M2 Precursor | Temp. (°C) | Time (Hours) | Catalyst Amount (mg) | Glucaric Acid Yield (%) |
|---|---|---|---|---|---|---|---|
| 1 | 1.5% Pt 1.5% Au / Titania [Süd Chemie 02-10] | -- | Pt(NO₃)₂ | 100 | 5 | 8 | 55 |
| 2 | 4% Pt 4% Au / Titania NorPro ST 61120 | HAuCl₂ | Pt(NO₃)₂ | 100 | 5 | 8 | 32 |

## Claims

1. A catalyst composition comprising particles of gold-platinum alloy and particles of platinum (0) on a support selected from the group consisting of titanias, zirconias, silicas and montmorillonites, wherein (a) the molar ratio of platinum to gold on the support is from 10:1 to 1:2, and (b) the particle sizes of the platinum (0) particles are substantially in the range of from 5 to 30 nanometers.

2. The catalyst composition of claim 1, wherein the gold-platinum alloy is formed at a temperature in the range of 200°C to 400°C.

3. The catalyst composition of claim 1 or 2, wherein the support is selected from the group consisting of titanias, zirconias and silicas.

4. The catalyst composition of any of claims 1 to 3, wherein the support comprises titania.

5. The catalyst composition of any of claims 1-4 wherein the total metal loading of the catalyst, excluding any metal that is from the support, is from 1 to 8 wt% .

6. The catalyst composition of any of claims 1-5 wherein the particles sizes of the platinum particles are substantially in the range of from 5 to 20 nanometers.

7. The catalyst composition of any of claims 1-6 wherein the particle sizes of the particles comprising gold are substantially in the range of from 2 to 20 nanometers.

8. The catalyst composition of any of claims 1-7 wherein at least 50% of the platinum particles have a size in the range of from 8 to 12 nanometers.

9. The catalyst composition of any of claims 1-8 wherein at least 50% of the gold particles have a size in the range of from 5 to 12 nanometers.

10. The catalyst composition of any of claims 1-9 wherein the total metal loading of the catalyst, excluding any metal that is from the support, is from 2 to 4 wt% .

11. The catalyst composition of any one of claims 1-10 wherein the molar ratio of platinum to gold is in the range of from 3:1 to 1:2.

12. The catalyst composition of any one of claims 1-11 wherein the molar ratio of platinum to gold is in the range of from 2:1 to 1:2.

13. The catalyst composition of any one of claims 1-12 wherein the molar ratio of platinum to gold is in the range of from 2:1 to 1:1.

14. A process for preparing glucaric acid comprising reacting glucose with a source of oxygen in the presence of the catalyst composition of any one of claims 1-13 and in the substantial absence of added base.

## Patentansprüche

1. Katalysatorzusammensetzung, die Teilchen aus einer Gold-Platin-Legierung und Teilchen aus Platin (0) auf einem Träger umfasst, der aus einer Gruppe ausgewählt ist, die aus Titan, Zirkoniumdioxid, Siliciumdioxid und Montmorilloniten besteht, wobei (a) das Molverhältnis von Platin zu Gold auf dem Träger 10:1 bis 1:2 beträgt und (b) die Teilchengrößen der Platinteilchen (0) im Wesentlichen im Bereich von 5 bis 30 Nanometer liegen.

2. Katalysatorzusammensetzung nach Anspruch 1, wobei die Gold-Platin-Legierung bei einer Temperatur im Bereich von 200 °C bis 400 °C gebildet wird.

3. Katalysatorzusammensetzung nach Anspruch 1 oder 2, wobei der Träger aus einer Gruppe ausgewählt ist, die aus Titan, Zirkoniumdioxid und Siliciumdioxid besteht.

4. Katalysatorzusammensetzung nach einem der Ansprüche 1 bis 3, wobei der Träger Titanoxid umfasst.

5. Katalysatorzusammensetzung nach einem der Ansprüche 1 bis 4, wobei die Gesamtmetallbeladung des Katalysators, ausgenommen jegliches Metall, das vom Träger stammt, 1 bis 8 Gew.-% beträgt.

6. Katalysatorzusammensetzung nach einem der Ansprüche 1 bis 5, wobei die Teilchengrößen der Platinteilchen im Wesentlichen im Bereich von 5 bis 20 Nanometern liegen.

7. Katalysatorzusammensetzung nach einem der Ansprüche 1 bis 6, wobei die Teilchengrößen der goldhaltigen Teilchen im Wesentlichen im Bereich von 2 bis 20 Nanometern liegen.

8. Katalysatorzusammensetzung nach einem der Ansprüche 1 bis 7, wobei mindestens 50 % der Platinteilchen eine Größe im Bereich von 8 bis 12 Nanometern aufweisen.

9. Katalysatorzusammensetzung nach einem der Ansprüche 1 bis 8, wobei mindestens 50 % der Goldteilchen eine Größe im Bereich von 5 bis 12 Nanometern aufweisen.

10. Katalysatorzusammensetzung nach einem der Ansprüche 1 bis 9, wobei die Gesamtmetallbeladung des Katalysators, ausgenommen jegliches Metall, das vom Träger stammt, 2 bis 4 Gew.-% beträgt.

11. Katalysatorzusammensetzung nach einem der Ansprüche 1-10, wobei das Molverhältnis von Platin zu Gold im Bereich von 3:1 bis 1:2 liegt.

12. Katalysatorzusammensetzung nach einem der Ansprüche 1-11, wobei das Molverhältnis von Platin zu Gold im Bereich von 2:1 bis 1:2 liegt.

13. Katalysatorzusammensetzung nach einem der Ansprüche 1-12, wobei das Molverhältnis von Platin zu Gold im Bereich von 2:1 bis 1:1 liegt.

14. Verfahren zur Herstellung von Glucarsäure, umfassend das Reagieren von Glucose mit einer Sauerstoffquelle in Gegenwart der Katalysatorzusammensetzung nach einem der Ansprüche 1 bis 13 und im Wesentlichen in Abwesenheit einer zugesetzten Basis.

## Revendications

1. Composition de catalyseur comprenant des particules d'alliage or-platine et des particules de platine (0) sur un support choisi dans le groupe constitué par les oxydes de titane, les zircones, les silices et les montmorillonites, dans laquelle (a) le rapport molaire du platine à l'or sur le support est de 10:1 à 1:2, et (b) les tailles de particule des particules de platine (0) sont sensiblement dans la plage de 5 à 30 nanomètres.

2. Composition de catalyseur selon la revendication 1, dans laquelle l'alliage or-platine est formé à une température dans la plage de 200 °C à 400 °C.

3. Composition de catalyseur selon la revendication 1 ou 2, dans laquelle le support est choisi dans le groupe constitué par les oxydes de titane, les zircones et les silices.

4. Composition de catalyseur selon l'une quelconque des revendications 1 à 3, dans laquelle le support comprend de l'oxyde de titane.

5. Composition de catalyseur selon l'une quelconque des revendications 1 à 4, dans laquelle la charge totale en métal du catalyseur, à l'exclusion de tout métal provenant du support, est de 1 à 8 % en poids.

6. Composition de catalyseur selon l'une quelconque des revendications 1 à 5, dans laquelle les tailles de particules des particules de platine sont sensiblement dans la plage de 5 à 20 nanomètres.

7. Composition de catalyseur selon l'une quelconque des revendications 1 à 6, dans laquelle les tailles de particule des particules comprenant de l'or sont sensiblement dans la plage de 2 à 20 nanomètres.

8. Composition de catalyseur selon l'une quelconque des revendications 1 à 7, dans laquelle au moins 50 % des particules de platine ont une taille dans la plage de 8 à 12 nanomètres.

9. Composition de catalyseur selon l'une quelconque des revendications 1 à 8, dans laquelle au moins 50 % des particules d'or ont une taille dans la plage de 5 à 12 nanomètres.

10. Composition de catalyseur selon l'une quelconque des revendications 1 à 9, dans laquelle la charge totale en métal du catalyseur, à l'exclusion de tout métal provenant du support, est de 2 à 4 % en poids.

11. Composition de catalyseur selon l'une quelconque des revendications 1 à 10, dans laquelle le rapport molaire du platine à l'or est dans la plage de 3:1 à 1:2.

12. Composition de catalyseur selon l'une quelconque des revendications 1 à 11, dans laquelle le rapport molaire du platine à l'or est dans la plage de 2:1 à 1:2.

13. Composition de catalyseur selon l'une quelconque des revendications 1 à 12, dans laquelle le rapport molaire du platine à l'or est dans la plage de 2:1 à 1:1.

14. Procédé de préparation d'acide glucarique comprenant la réaction de glucose avec une source d'oxygène en présence de la composition de catalyseur selon l'une quelconque des revendications 1 à 13 et en l'absence substantielle de base ajoutée.
